# EUROPEAN PATENT APPLICATION

(11) **EP 4 104 872 A1**
(43) Date of publication of application: **21.12.2022**
(21) Application number: 22176574.6
(22) Date of filing: 31.05.2022
(51) Int. Cl.: A61L 27/02, A61L 27/54, A61L 27/58

(54) **IMPLANTABLE PHARMACEUTICAL COMPOSITION PREPARED FROM COMPONENTS CONSISTING ONLY OF CALCIUM SULFATE ALPHA-HEMIHYDRATE, VANCOMYCIN AND TOBRAMYCIN**

(30) Priority: 18.06.2021 GB 202108729
(71) Applicant: Biocomposites Limited, Keele, Staffordshire ST5 5NL (GB)
(72) Inventor: Aiken, Sean Sydney, Staffordshire, ST5 5NL (GB); Laycock, Phillip Anthony, Staffordshire, ST5 5NL (GB); Cooper, John Joseph, Staffordshire, ST5 5NL (GB); Bratt, John Stephen, Staffordshire, ST5 5NL (GB); Harris, Michael Christopher James, Staffordshire, ST5 5NL (GB)
(74) Representative: Wilson Gunn

(57) **Abstract**

The present invention concerns a composition for an implantable pharmaceutical composition prepared from components consisting only of calcium sulfate α-hemihydrate in combination with two antibiotics, vancomycin and tobramycin, for the treatment of infection in bone and soft tissue.

## Description

### FIELD OF THE INVENTION

The present invention concerns a pharmaceutical composition for delivery of an implantable material/drug/drug device combination prepared from components consisting only of calcium sulfate α-hemihydrate in combination with two antibiotics, vancomycin and tobramycin, which are provided in the forms of vancomycin hydrochloride and tobramycin sulfate, for the treatment or prophylaxis of infection in bone and soft tissue. Both sets of terms will be used herein, and they are considered to be interchangeable with each other.

The composition is typically prepared for implantation as absorbable beads into infected surgical sites in both bone and soft tissue. In addition, the invention relates to a method of formation of the aforementioned composition, which may be in the form of beads or pellets, and to the resultant physical properties of the composition when combined at a specific dose/weight combination.

### BACKGROUND OF THE INVENTION

Studies of the use of calcium sulfate bone void fillers in conjunction with antibiotics for direct introduction into an infected surgical site, have shown encouraging results. In addition to facilitating the regeneration of bone, they provide high local levels of antibiotic, thus also offering the beneficial effect of filling the dead space left by debridement.

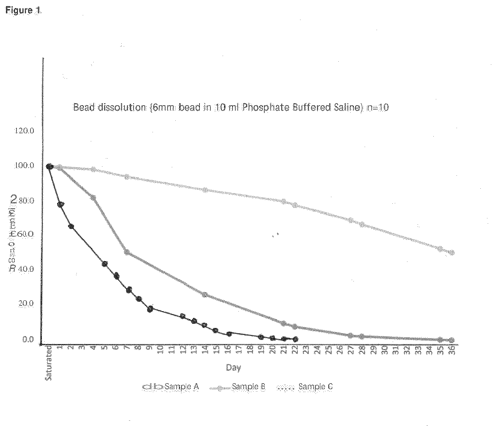

The dissolution rate of calcium sulfate can vary greatly as a result of differing manufacturing processes for the calcium sulfate. The addition of varying quantities of calcium sulfate dihydrate and/or potassium sulfate to the hemihydrate powder is often used to accelerate the setting times of calcium sulfate beads or pellets, but this can lead to differences in porosity and pore size which can make the dissolution rate variable to a significant degree. The graph depicted in Fig. 1 shows the variation in dissolution between two commercially available calcium sulfates and the calcium sulfate α-hemihydrate used in the present invention, which each have different methods of manufacture.

A pharmaceutical grade of calcium sulfate material, suitable for use as a carrier of therapeutic agents to the body, has the levels of purity demanded by the pharmaceutical and medical device industries. It is often used as an excipient in tablet formulations. However, this high purity calcium sulfate material, which is generally produced by chemical precipitation reactions utilising reagents having the necessary high levels of purity, and is presented as the dihydrate form, has a fine particle size and high specific surface area, and as such, when treated by heat (either 'wet' or 'dry') to give an α-hemihydrate form of calcium sulfate which has a high water demand.

It is therefore required to be mixed with a relatively large quantity of water containing the therapeutic agents, as much as a liquid:powder ratio of 1:1 by weight, in order to form a paste consistency suitable for incorporation of therapeutical reagents for implantation. The powder in the liquid:powder ratio refers to the calcium sulfate material, while the liquid refers to the aqueous solution containing the therapeutic agents/antibiotics. This gives a set, dihydrate form having a high porosity, low strength and low density and thus a rapid rate of dissolution within the body of a patient. This results in too rapid a release of the incorporated therapeutic agents/antibiotics. This situation in certain patients and surgical procedures may result in adverse events such as hypercalcaemia and acute kidney injury to the patient.

In addition, many commercially available forms of calcium sulfate α-hemihydrate contain additional excipients such as calcium sulfate dihydrate, potassium sulfate, trisodium phosphate and sodium phosphate to accelerate or slow down the setting time, or calcium stearate to slow down the absorption rate. Therefore, such materials cannot be considered to be a pure calcium sulfate.

It is desired to overcome the aforementioned problems, and the present invention achieves this.

The present invention therefore provides a pharmaceutical composition prepared from components consisting only of:
a) 18.4g pharmaceutical grade, phase pure calcium sulfate α-hemihydrate;
b) 1000 mg of vancomycin hydrochloride; and
c) 240 mg of tobramycin sulfate;
wherein the calcium sulfate α-hemihydrate is prepared by first converting a source of pharmaceutical grade calcium sulfate dihydrate to soluble calcium sulfate anhydrite by hydrothermal treatment in suspension, and then converting the resulting soluble anhydrous calcium sulfate to calcium sulfate α-hemihydrate by the following steps:
i) adding the calcium sulfate dihydrate to a quantity of water in a water:calcium sulfate dihydrate ratio of 0.3:1-0.5:1 to form a suspension and autoclaving the suspension at 235-265°C for 1-2 hours to dehydrate to form soluble calcium sulfate anhydrite;
ii) allowing the soluble calcium sulfate anhydrite to cool in the autoclave in suspension to rehydrate back to calcium sulfate dihydrate; and
iii) draining off excess water and autoclaving the rehydrated calcium sulfate dihydrate at 110-150°C for 1-2 hours to convert the rehydrated calcium sulfate dihydrate to calcium sulfate α-hemihydrate.

Once the calcium sulfate α-hemihydrate has been formed in step (iii), it is typically ground to a powder for further processing.

The water:calcium sulfate dihydrate ratio in step (i) may typically be in the range of 0.4:1-0.45-1, such as between about 0.42:-0.43:1.

The autoclaving in step (i) may typically be carried out at about 250°C for about 90 minutes; while the autoclaving in step (iii) may typically be carried out at about 130°C for about 90 minutes.

The vancomycin hydrochloride is typically added in the form of a powder and the tobramycin sulfate is typically added in the form of a liquid. Combining the liquid tobramycin sulfate with vancomycin hydrochloride powder forms a suspension containing vancomycin and tobramycin.

The above-described method provides a low water demand (pharmaceutical grade) calcium sulfate α-hemihydrate. The liquid:powder ratio necessary then to form a 'paste' consistency suitable for incorporating the vancomycin hydrochloride and tobramycin sulfate into the calcium sulfate α-hemihydrate would ideally be in the range 0.26-0.35:1, more typically 0.28-0.32:1 parts by weight, *i.e.* the solution required to hydrate the calcium sulfate α-hemihydrate when forming beads containing the vancomycin hydrochloride and tobramycin sulfate. The powder in the liquid:powder ratio herein refers to the calcium sulfate α-hemihydrate, while the liquid refers to the suspension containing vancomycin and tobramycin antibiotics. By way of comparison, an alternative calcium sulfate which is commercially available has a higher water demand of 0.39:1, which results in a higher porosity, lower strength and lower density and thus a more rapid rate of dissolution within the body of a patient. This is undesirable.

The calcium sulfate α-hemihydrate requires a water demand (or solution) in the defined range to fully convert to dihydrate, *i.e.* set hard. If the amount of solution required is too high, then the porosity is increased and therefore the dissolution rate will be too high. Too low a water demand will compromise strength. As used herein, the "solution" is liquid tobramycin combined with vancomycin, which forms a suspension containing vancomycin and tobramycin.

Vancomycin hydrochloride is a tricyclic glycopeptide antibiotic which inhibits the synthesis of bacterial cell wall membranes in susceptible bacteria. Its spectrum is limited to the treatment of infections caused by aerobic and anaerobic gram-positive bacteria, including *Staphylococcus aureus and Staphylococcus epidermidis* (including methicillin-resistant strains) and Streptococcus Species. Vancomycin is not effective on gram negative strains because the outer membrane of this group of bacteria contains a peptidoglycan polymer layer impermeable to the drug.

Tobramycin sulfate is an aminoglycoside antibiotic. Its mode of action is by binding to 30S subunit bacterial ribosomes. This impairs the proofreading of t-RNA resulting in the production of faulty proteins that insert into the cytoplastic membrane, killing the bacteria. Tobramycin is effective against Gram positive *Staphylococcus aureus* and against a number of Gram-negative bacteria including *Pseudomomas aeruginosa.*

For some serious infections, the efficacy of vancomycin hydrochloride is not always satisfactory. The rise of vancomycin resistant strains is also concerning. The combination of vancomycin hydrochloride with tobramycin sulfate has shown promising results in-vitro and has also been demonstrated clinically.

Porosity is an important characteristic of a drug eluting material. There is a direct correlation between pore size and dissolution rate. The more porous the material, the more fluids can penetrate the material, accelerating the rate of dissolution and therefore allowing the contained medicaments to be released. The porosity of the material is determined by the purity of the calcium sulfate α-hemihydrate, the speed of crystallisation to dihydrate (*i.e.* the setting time) also the dose of the active pharmaceutical ingredients. In addition, the volume of aqueous solution containing the active pharmaceutical ingredients - *i.e.* the vancomycin and tobramycin - is critical to the final composition of the set beads. Adding too little of the aqueous solution results in only a partial conversion to calcium sulfate dihydrate, producing a weaker, more soluble biphasic material. Conversely, too much liquid results in surplus water for crystallisation which will not take part in the reaction and remains as free water in the material, contributing to the subsequent porosity. The liquid to powder ratio is therefore an important parameter to produce a finished material with the ideal surface area and pore size, critical factors in the dissolution of the beads and consequently the elution of the added medicaments.

Porosity and surface area of a material can be measured by the Brauner, Emmett and Teller (BET) technique or alternatively by mercury intrusion porosimetry. The BET technique involving nitrogen adsorption is widely used for detecting open pores mainly in the micro (≤ 2 nm) to mesopore (2-50 nm) region. The volume of nitrogen adsorbed to the surface of the material is measured at the boiling point of nitrogen (-196°C). At this temperature the nitrogen gas is below the critical temperature and so condenses on the surface in a monolayer. As the size of the nitrogen atom/molecule is known, the amount of adsorbed (condensed) gas is correlated to the total surface area of the particles including pores at the surface.

Average pore size distribution can be deduced according to the Barrett-Joyner-Halenda (BJH) method. Where BET theory measures the specific surface area of materials, BJH theory is a pore size distribution determination method. This method determines pore area and specific pore volume using adsorption and desorption techniques and characterizes pore size distribution independent of external area due to particle size of the sample.

Setting time of the material can be determined by a variety of methods. The surface penetration test involves applying a weighted Vicat or Gilmore needle to the surface of the material. The material is deemed to be set when it has either penetrated to a given depth (Vicat) or developed sufficient strength to support the needle and it no longer makes an indentation (Gilmore). Both Vicat and Gilmore tests are the subject of multiple standards, including ASTM. An alternative method is acoustic monitoring where the sound frequencies produced when dropping the material onto a hard surface from a set height are recorded. Set time is confirmed when the frequencies match those of a control sample that is fully set. A more accurate method for determining set time is by monitoring the temperature of the setting mass by use of a calibrated thermocouple inserted into the material. The calcium sulfate crystallisation reaction is exothermic, and the mass will continue to increase in temperature until the reaction has completed, at which point the material will start to reduce in temperature. The peak of the temperature curve indicates the final set time.

The present invention thus provides an implantable composition for treatment of localised infections in the body by providing a dual antibiotic loaded material produced from materials consisting only of a pharmaceutically pure calcium sulfate α-hemihydrate, in combination with two antibiotics, vancomycin and tobramycin, in particular amounts, together providing a synergistic effect.

The present invention provides a composition that not only provides a local delivery carrier for the two antibiotics but has specific properties necessary to provide a controlled reproducible dissolution rate. Pore size, surface area and compressive strength all determine the physical properties of the set beads in respect to the dissolution rate and elution kinetics of the two antibiotics. The purity of the calcium sulfate free of setting aids and other processing excipients in combination with a fixed dose of two antibiotics which together target a wider range of bacterial infections is described.

### DETAILED DESCRIPTION OF THE INVENTION

The present invention provides a composition prepared from components consisting of 18.4g pharmaceutical grade, phase pure calcium sulfate α-hemihydrate, 1000 mg of vancomycin hydrochloride; and 240 mg of tobramycin sulfate; where the calcium sulfate α-hemihydrate is prepared using the steps (i)-(iii) discussed above. The composition is typically provided in the form of beads or pellets, which are formed by setting amounts of a paste of the composition within a mould mat containing cavities of a specific size and shape to produce cylindrical beads with one hemispherical end for implantation into a surgical site.

The calcium sulfate used to prepare the composition is calcium sulfate α-hemihydrate. The composition of the invention is prepared using only calcium sulfate α-hemihydrate, vancomycin hydrochloride and tobramycin sulfate, and is free of processing and setting aids including calcium sulfate dihydrate, potassium phosphate, sodium chloride, sodium sulfate or calcium stearate. This simplifies the process, makes it less expensive, and reduces the variability in porosity and pore size of the composition.

The vancomycin is provided in the form of its hydrochloride salt. The vancomycin hydrochloride will typically be in powder form.

The tobramycin is provided in the form of its sulfate salt. The tobramycin sulfate will typically be in a solution and used for both solubilising and suspending the vancomycin hydrochloride powder and hydrating the calcium sulfate hemihydrate to form set beads or pellets. The volume of solution typically used to hydrate the α-hemihydrate is 6 ml and represents a water powder ratio of 0.32.

If the composition is in the form of beads or pellets, they are formed by measuring the tobramycin sulfate solution at the appropriate dose and adding the solution to the specified weight of vancomycin hydrochloride powder. The vancomycin hydrochloride partly dissolves in the tobramycin sulfate solution to produce a suspension containing the two antibiotics. The suspension is then applied to the specified weight of calcium sulfate α-hemihydrate powder and mixed, typically for about 20 seconds. The resultant paste and then applied to a bead mould and allowed to set.

Once set, the resultant beads are then removed from the mould and are ready for implantation into the surgical site. The beads possess a specific surface area and pore size distribution allowing for a predictable and controlled rate of dissolution and drug elution when measured *in vitro.* In one specific embodiment, the beads are cylindrical, having a diameter between 2.8 mm and 3.2 mm at their widest point. One end of the bead is hemispherical. The bead mould will typically contain a total of 837 cavities to incorporate all the paste.

The bead mould is typically a two-sided mould with bead cavities on both sides. This facilitates easier removal of the beads by adding flexibility to the mould. The cavities are typically identical on both sides. In one specific embodiment, the bead mould is square in shape having sides of 152.5 mm and a depth of 7.5 mm.

The physical properties of the beads are a product of the antibiotic doses and the weight of the calcium sulfate hemihydrate powder used to make them. The ratio of drug concentration to hemihydrate powder (*i.e.* the liquid:powder ratio referenced above) is critical to the performance of the beads once implanted. The liquid:powder ratio is linked to bead set time, compressive strength and elution profile of both pharmaceutical ingredients.

As discussed above, the porosity of the composition is determined in part by the volume of aqueous solution containing the active pharmaceutical ingredients. Adding too little of the aqueous solution to the powder results in only a partial conversion to calcium sulfate dihydrate, producing a weaker, more soluble biphasic material. Conversely, too much liquid results in surplus water for crystallisation which will not take part in the reaction and remains as free water in the material, contributing to the subsequent porosity and leaving a weaker material. The liquid to powder ratio is an important parameter to produce a finished material with the ideal surface area and pore size, critical factors in the dissolution of the beads and consequently the elution of the added pharmaceutical ingredients.

The ideal liquid to powder ratio to fully convert the calcium sulfate α-hemihydrate is in the range of 0.26-0.35:1, more typically 0.28-0.32:1. The liquid to powder ratio of other commercially available calcium sulfate products use ratios which are either lower or higher than this, as shown in Table 1 below, due to them being manufactured in different ways to the calcium sulfate α-hemihydrate used in the present invention.

In one specific embodiment, the dose of tobramycin sulfate in solution is 40 mg/ml. 6 ml of solution is used to partially dissolve the vancomycin hydrochloride at a dose of 1000 mg and the resultant aqueous suspension contains 166.66 mg/ml of vancomycin hydrochloride. This will fully hydrate 18.4g of calcium sulfate α-hemihydrate powder. The addition of the extra weight of powders to the calcium sulfate α-hemihydrate does not require additional solution as the antibiotics do not take part in the setting reaction.

In the specific embodiment of 18.4g calcium sulfate α-hemihydrate with 1000g vancomycin and 240 mg tobramycin prepared as described, the set beads have specific properties in terms of BET surface area of between about 0.2 m²/g and 0.8 m²/g; more typically between about 0.4 m²/g and 0.6 m²/g.

According to one embodiment of the invention, the beads or pellets have a BJH adsorption average pore diameter (4v/A) area deduced according to the Barrett-Joyner-Halenda (BJH) pore size distribution method between about 10 nm and 25 nm.

The beads when produced according to the aforementioned embodiments, have a setting time of under 20 minutes as defined as the time required from the combination of the aqueous suspension to the calcium sulfate alpha-hemihydrate until hardening is achieved. The disclosed embodiments produce antibiotic loaded calcium sulfate dihydrate beads that maintain sufficient compressive strength that is not compromised by the addition of antibiotics. According to one embodiment, the compressive strength is between 10 and 20 MPa at 1 hour after mixing.

The beads produced using the aforementioned method may be used in surgical procedures as part of an infection management strategy where they absorb and elution the full amount of vancomycin and tobramycin at the surgical site reducing the risk of systemic toxicity compared to systemic administration of the two antibiotics.

### BRIEF DESCRIPTION OF THE FIGURES

Figure 1 illustrates the variation in dissolution between three commercially available calcium sulfates with different liquid/powder ratios as shown in table 1:

**Table 1. 3 Commercially available calcium sulfates with different liquid/powder ratios**

| | **Liquid/Powder Ratio** |
|---|---|
| Sample 1 (Synthecure) | 0.240:1 |
| Sample B (the calcium sulfate α-hemihydrate of the invention) | 0.320:1 |
| Sample C (InterSep^{®}) | 0.395:1 |

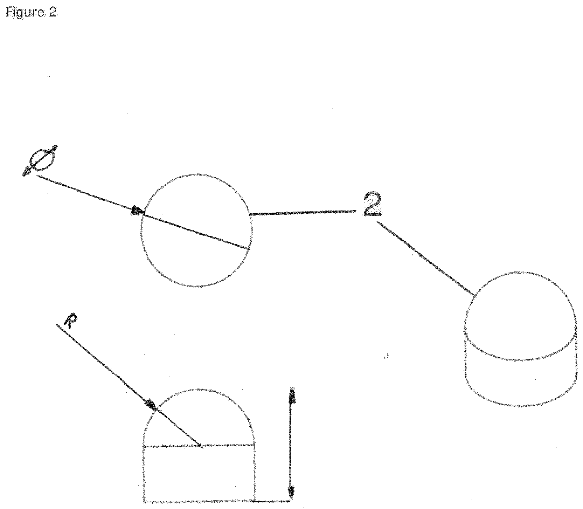

Figure 2 shows a plan view, sectional side view and perspective view of a 3 mm bead 2 made of the composition of the invention. The diameter of the bead at its widest point is 3mm.

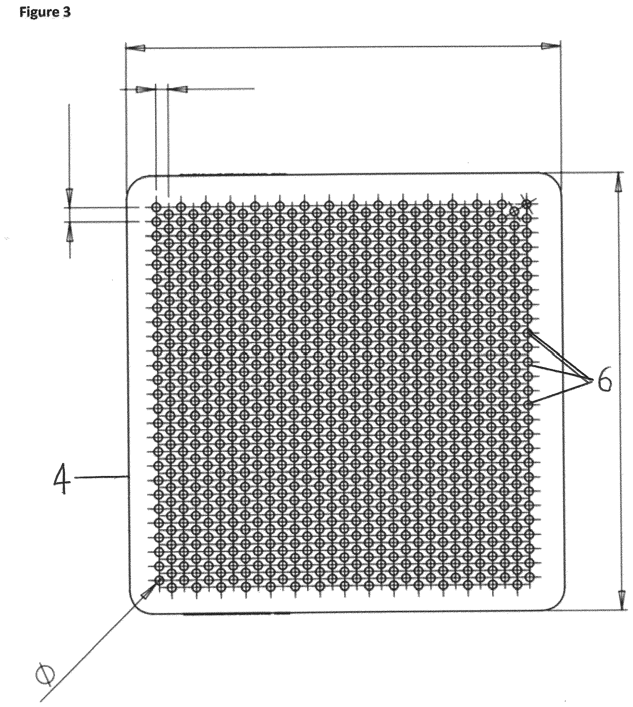

Figure 3 shows a plan view of a mould 4 used to make the beads. The mould has a plurality of cavities 6 thereon, which here each have a diameter of 3 mm.

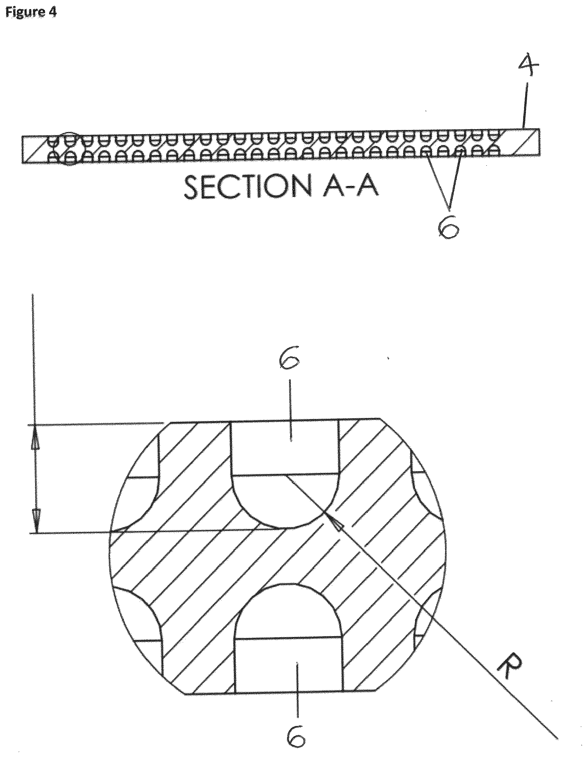

The bead mould 4 is typically a two-sided mould with bead cavities 6 on both sides, as shown in Figure 4, which shows two sectional side views of the bead mould 4, one with a wider perspective, and the other showing two corresponding cavities 6 on each side of the mould. Having the cavities 6 on both sides of the mould 4 facilitates easier removal of the beads by adding flexibility to the mould 4. The cavities are identical on both sides of the mould 4.

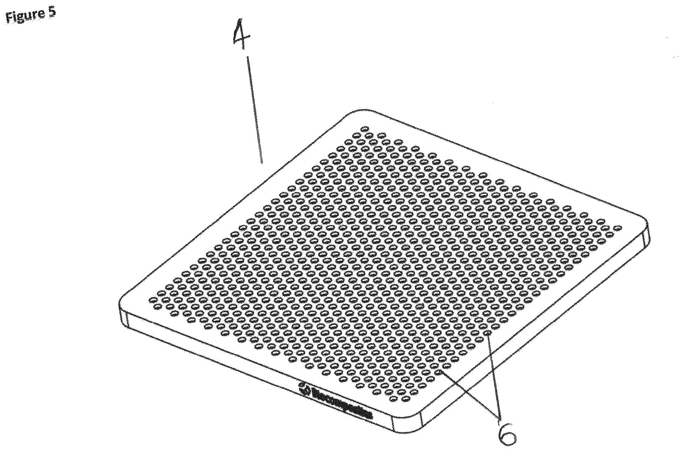

Figure 5 shows another perspective view of the bead mould 4, showing the mould with its arrangement of cavities 6.

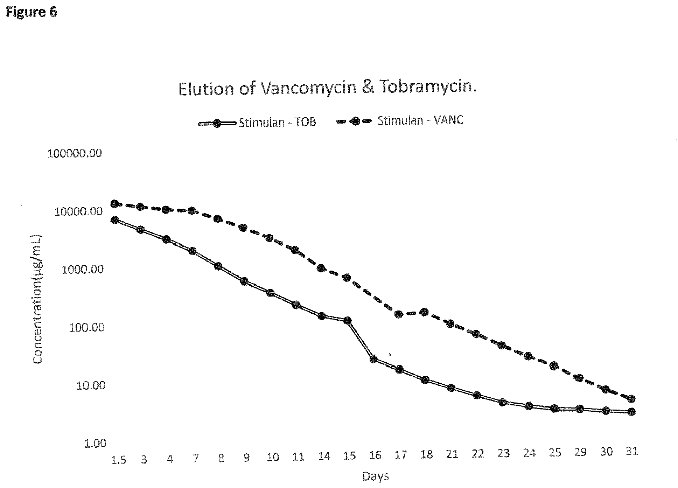

Figure 6 shows typical elution profiles of vancomycin hydrochloride and tobramycin sulfate from the composition as analysed by liquid chromatography mass spectrometry with 1.5g of beads in 4 ml of phosphate buffered saline (PBS). Partial solution exchange (1.5 ml) was performed at 24 hour periods and replenished with fresh PBS.

### EXAMPLES

### EXAMPLE 1

### Demonstrate surface area

18.4 g of calcium sulfate α-hemihydrate was combined with 240 mg (6 ml) of tobramycin sulfate liquid with 1000 mg of vancomycin hydrochloride powder and formed into 3 mm beads and allowed to set. A sample set of 3 beads were selected at random for analysis. The beads were vacuum degassed at room temperature and then isotherms were obtained using a nitrogen adsorption instrument (Tristar II Plus. Micromeritics Ltd.) Results are shown in Table 2.

**Table 2. BET surface area of three lots of calcium sulfate combined with vancomycin and tobramycin**

| **Sample** | **BET Surface Area (m²/g)** |
|---|---|
| Lot 1 Sample 1 | 0.356 |
| Lot 1 Sample 2 | 0.364 |
| Lot 1 Sample 3 | 0.372 |
| Lot 2 Sample 1 | 0.272 |
| Lot 2 Sample 2 | 0.256 |
| Lot 2 Sample 3 | 0.280 |
| Lot 3 Sample 1 | 0.254 |
| Lot 3 Sample 2 | 0.267 |
| Lot 3 Sample 3 | 0.275 |

### EXAMPLE 2

### Demonstrate BJH pore size distribution

18.4 g of calcium sulfate α- hemihydrate was combined with 240 mg (6 ml) of tobramycin sulfate liquid with 1000 mg of vancomycin hydrochloride powder and formed into 3mm beads and allowed to set. A sample set of 3 beads were selected at random for analysis. The beads were vacuum degassed at room temperature and then isotherms were obtained using a nitrogen adsorption instrument (Tristar II Plus. Micromeritics Ltd.) Results are shown in Table 3.

**Table 3. BJH Adsorption average pore diameter of three lots of calcium sulfate combined with vancomycin and tobramycin**

| **Sample** | **BJH Adsorption Average Pore Diameter (4V/A) (nm)** |
|---|---|
| Lot 1 Sample 1 | 15.2249 |
| Lot 1 Sample 2 | 14.9519 |
| Lot 1 Sample 3 | 14.2940 |
| Lot 2 Sample 1 | 21.5225 |
| Lot 2 Sample 2 | 22.3880 |
| Lot 2 Sample 3 | 20.2697 |
| Lot 3 Sample 1 | 19.9426 |
| Lot 3 Sample 2 | 18.9352 |
| Lot 3 Sample 3 | 17.2245 |

### EXAMPLE 3

### Elution data for VT

18.4 g of calcium sulfate α-hemihydrate was combined with 240 mg (6 ml) of tobramycin sulfate liquid with 1000 mg of vancomycin hydrochloride powder and formed into 3mm beads and allowed to set. Three grams of beads were immersed in 4 mL of sterile phosphate-buffered saline (PBS) at 37°C. At each time point, 1.5ml of the solution was sampled and replenished with fresh PBS. The samples were then syringe filtered using a 0.2 µm 13mm PES syringe filter into 2mL amber glass vials.

Quantification of eluted antibiotics was measured using a single quadrupole mass spectrometer (MS) coupled to a 1260 infinity II series liquid chromatography stack (LCMS) (Agilent Technologies). Depending on the concentration, samples were analysed either neat or diluted to ensure values fell within the linear range of the standard curves. Concentrations were calculated from peak areas using standard curves developed from dilutions of antibiotic prepared to known concentrations. Separation was conducted with a Poroshell 120-SB-C18 column (21. X 100 mm, 2.17 µm).

LCMS conditions were Buffer A was 99.9% H2O, 0.1% Heptafluorobutyric Acid and B was 99.9% Acetonitrile (CAN), 0.1% % Heptafluorobutyric Acid. The gradient was held with 30.0% B for 2 min before increasing linearly to 95.0% over 5 min. The 100% B was maintained for a further 1 min before rapid equilibration in 100% A. The flow rate was constant at 0.3 mL/min.

The gas temperature in the source was 325°C with a flow rate of 9 L/min. The nebulizer pressure was 35 pounds per square inch and the capillary voltage 3.5 kV. Fragmentor and collision energy voltages were optimized for each antibiotic. Eluted concentrations are shown in Table 4 and Figure 6.

**Table 4. Elution concentrations**

| | Tobramycin | Vancomycin |
|---|---|---|
| Day | µg/ml | µg/ml |
| 1 | 7046.208 | 13420.69 |
| 3 | 4693.264 | 11787.92 |
| 4 | 3130.589 | 10336.16 |
| 7 | 1972.743 | 9807.795 |
| 8 | 1058.751 | 6936.349 |
| 9 | 574.1567 | 4778.238 |
| 10 | 354.742 | 3093.854 |
| 11 | 216.352 | 1932.859 |
| 14 | 135.2563 | 905.3717 |
| 15 | 109.8367 | 603.5953 |
| 17 | 15.186 | 135.9353 |
| 18 | 9.977667 | 147.697 |
| 21 | 7.125 | 91.762 |
| 22 | 5.193667 | 59.85067 |
| 23 | 3.865667 | 36.98767 |
| 24 | 3.277 | 23.549 |
| 25 | 2.913 | 16.01967 |
| 29 | 2.833 | 9.746 |
| 30 | 2.598 | 6.082333 |
| 31 | 2.453667 | 4.065667 |

The results demonstrate a controlled and reproducible elution profile with concentrations exceeding the minimally inhibitory concentrations (MIC) for most out to 31 days as demonstrated in this in-vitro experiment. Local concentrations are achieved higher than those which could safely be administered systemically.

## Claims

1. A pharmaceutical composition prepared from components consisting only of:
a) 18.4 g pharmaceutical grade, phase pure calcium sulfate α-hemihydrate;
b) 1000 mg of vancomycin hydrochloride; and
c) 240 mg of tobramycin sulfate;
wherein the calcium sulfate α-hemihydrate is prepared by first converting a source of pharmaceutical grade calcium sulfate dihydrate to soluble calcium sulfate anhydrite by hydrothermal treatment in suspension, and then converting the resulting soluble anhydrous calcium sulfate to calcium sulfate α-hemihydrate by the following steps:
i) adding the calcium sulfate dihydrate to a quantity of water in a water:calcium sulfate dihydrate ratio of 0.3:1-0.5:1 to form a suspension and autoclaving the suspension at 235-265°C for 1-2 hours to dehydrate to form soluble anhydrite;
ii) allowing the soluble anhydrite to cool in the autoclave in suspension to rehydrate back to calcium sulfate dihydrate;
iii) draining off excess water and autoclaving again at 110-150°C for 1-2 hours to convert the rehydrated calcium sulfate dihydrate to calcium sulfate α-hemihydrate.

2. The composition according to claim 1, wherein the vancomycin hydrochloride and tobramycin sulfate are combined to form a suspension, and the suspension is combined with the calcium sulfate α-hemihydrate in powder form in a liquid:powder ratio of between 0.26 and 0.35 parts by weight;
wherein the liquid:powder ratio is optionally between 0.28 and 0.32 parts by weight.

3. The composition according to any preceding claim, wherein the vancomycin hydrochloride is provided in a powder form; and/or
wherein the tobramycin sulfate is provided in a liquid form.

4. The composition according to any preceding claim, wherein the composition is in the form of beads or pellets;
wherein the beads or pellets are optionally between about 2.8 and 3.2mm in diameter at their widest point; and or
wherein the beads or pellets optionally have a compressive strength of between 10 MPa and 20 MPa at 1 hour after setting.

5. The composition according to claim 4, wherein the beads or pellets have a BET specific surface area obtained using the Brunauer Emmett-Teller (BET) Method of between about 0.2 m²/g and 0.8 m²/g.

6. The composition according to according to claim 4 or claim 5, wherein the beads or pellets have a BJH adsorption average pore diameter (4v/A) area deduced according to the Barrett-Joyner-Halenda (BJH) pore size distribution method between about 10 nm and 25 nm.

7. A method of manufacturing a composition according to any preceding claim, the method comprising the steps of:
a) mixing 1000 mg of vancomycin hydrochloride with 240 mg of tobramycin sulfate, the tobramycin sulfate being in the form of a liquid, to produce a suspension containing vancomycin and tobramycin;
b) combining said suspension with a powder consisting of 18.4g of pharmaceutical grade, phase pure calcium sulfate α-hemihydrate to form a mixture;
c) forming said mixture into beads or pellets which are allowed to set and solidify using a plurality of moulds; and
d) producing beads or pellets of the composition.

8. The method according to claim 7, wherein:
a) the vancomycin hydrochloride is provided in the form of a powder; and
b) the beads or pellets of the composition are individually shaped as a cylinder with a single hemispherical end.

9. The method according to claim 7 or claim 8, wherein in step (b), the suspension containing the vancomycin hydrochloride and tobramycin sulfate is combined with the calcium sulfate α-hemihydrate in powder form in a liquid:powder ratio of between 0.26 and 0.35 parts by weight;
wherein the liquid:powder ratio is optionally between 0.28 and 0.32 parts by weight.

10. The method according to any of claims 7-9, wherein the tobramycin is in the form of an aqueous solution;
wherein the concentration of tobramycin in the aqueous solution is optionally from about 25-50 mg/ml.

11. The method according to claim 10, wherein the volume of the aqueous solution to receive the vancomycin for a single dose of the composition is between about 5.5 ml and 7.5 ml.

12. The method according to any of claims 7-11, wherein the beads or pellets are prepared using a mould mat containing cavities wherein the size, spacing and geometric arrangement of cavities is such that any straight line drawn along the full length of the mold mat parallel to an edge of the mold mat in at least one direction and within the arrangement of cavities on the mat will always intersect at least one cavity;
wherein the mould mat optionally contains cavities on both sides of the mat.

13. The method according to any of claims 7-12, wherein the beads or pellets set in a time of between about 10 and 20 minutes as measured by the temperature rise method.

14. The method according to claim 13, wherein the setting time is determined by the insertion of a thermometer probe inside the prepared paste to track changes in temperature over time.

15. A composition according to any of claims 1-6 for the treatment of of infection in bone and soft tissue.
